**Europäisches Patentamt**

**European Patent Office**

**Office européen des brevets**

(11) Veröffentlichungsnummer: **0 217 231**
**A2**

(12) **EUROPÄISCHE PATENTANMELDUNG**

(21) Anmeldenummer: 86112829.6

(22) Anmeldetag: 17.09.86

(51) Int. Cl.⁴: **G01N 27/46**

(30) Priorität: 28.09.85 DE 3534616

(43) Veröffentlichungstag der Anmeldung:
08.04.87 Patentblatt 87/15

(84) Benannte Vertragsstaaten:
**DE FR GB SE**

(71) Anmelder: **Drägerwerk Aktiengesellschaft**
**Moislinger Allee 53-55**
**D-2400 Lübeck 1(DE)**

(72) Erfinder: **Hühmer, Siegfried, Dipl.-Ing.**
**Hamburger Strasse 211**
**D-2060 Bad Oldesloe(DE)**
Erfinder: **Mergenthaler, Peter**
**Wagnerstrasse 7**
**D-2958 Ostrhauderfehn(DE)**

(54) **Aufnahmevorrichtung für Gassensoren.**

(57) Eine Aufnahmevorrichtung zum Anschluß von Gassensoren an eine mit einer Begasungsöffnung versehene Meßgaskammer, in welche eine ein betätigbares Absperrelement enthaltende Zuführungsleitung für das Meßgas mündet und an deren Begasungsöffnung eine Sensorhalterung angeordnet ist, an welche ein bewegliches, in Richtung auf die Begasungsöffnung wirkendes Druckstück angreift, soll derart verbessert werden, daß beim Anbringen des Gassensors auf die Meßgaskammer gleichzeitig und ohne eine zusätzliche Betätigungshandlung die Zuführungsleitung für das Meßgas in die Meßgaskammer geöffnet und nach Entfernen des Gassensors aus der Aufnahmevorrichtung wieder geschlossen wird. Dazu ist vorgesehen, daß das Druckstück als Betätigungsteil (12) für das Absperrelement (14) der Zuführungsleitung (16) ausgebildet ist.

Fig 1

EP 0 217 231 A2

## Aufnahmevorrichtung für Gassensoren

Die Erfindung betrifft eine Aufnahmevorrichtung zum Anschluß von Gassensoren an eine mit einer Begasungsöffnung versehenen Meßgaskammer, in welche eine ein betätigbares Absperrelement enthaltende Zuführungsleitung für das Meßgas mündet und an deren Begasungsöffnung eine Sensorhalterung angeordnet ist, an welche ein bewegliches, in Richtung auf die Begasungsöffnung wirkendes Druckstück angreift.

Eine derartige Aufnahmevorrichtung ist in der EP-A 74 498 beschrieben.

Die bekannte Aufnahmevorrichtung besitzt eine Meßgaskammer, welche eine Begasungsöffnung aufweist, auf die der Gassensor mit seiner gassensitiven Meßfläche zentrisch aufgelegt wird. Eine Halteklammer als Druckstück wird auf die Rückseite des Gassensors geklemmt, so daß die gassensitive Meßfläche auf der Öffnung der Meßgaskammer aufliegt. Das Meßgas, welches in dem bekannten Falle zum Zwecke der Kalibrierung in die Meßgaskammer eingeleitet wird, wird aus getrennten Vorratsbehältern über Zuführleitungen in die Meßgaskammer geführt, nachdem über einen separaten Schalter die Zuführungsleitung von der Gasquelle zur Meßgaskammer geöffnet wurde.

Bei dem bekannten Gerät muß mit Sorgfalt die gassensitive Meßfläche des Gassensors genau auf die Begasungsöffnung der Meßgaskammer gelegt werden und ein entsprechender Spannhebel als Druckstück zur Fixierung des Gassensors auf die Rückseite des Gassensors aufgebracht werden. Außerdem muß mit einem getrennten Handgriff die Gaszuführungsleitung für das Meßgas geöffnet werden. Nach Abschluß der Messung bzw. der Kalibrierung kann der Gassensor von der Meßgaskammer entfernt werden, jedoch darf nicht vergessen werden, zusätzlich die Gaszuführungsleitung wieder abzusperren. Wenn dieses Absperren versäumt wird, entströmt weiterhin Meßgas aus der Gaszuführungsleitung über die Meßgaskammer ins Freie, wodurch der Vorrat an Meßgas in unkontrollierter Weise entweicht, möglicherweise ohne daß es der Benutzer bemerken kann.

Die Aufgabe der vorliegenden Erfindung ist darin zu sehen, eine Aufnahmevorrichtung zum Anschluß von Gassensoren an eine Meßgaskammer der genannten Art so zu verbessern, daß beim Anbringen des Gassensors auf die Meßgaskammer gleichzeitig und ohne eine zusätzliche Betätigungshandlung die Zuführungsleitung für das Meßgas in die Meßgaskammer geöffnet und nach Entfernen des Gassensors aus der Aufnahmevorrichtung wieder geschlossen wird.

Die Lösung der genannten Aufgabe besteht darin, daß das Druckstück als Betätigungsteil für das Absperrelement der Zuführungsleitung ausgebildet ist.

Der Vorteil der Erfindung liegt im wesentlichen darin, daß nunmehr die Zuführungsleitung nur dann geöffnet ist und das Meßgas in die Meßgaskammer leitet, solange der Gassensor in der Aufnahmevorrichtung gehalten ist. Ein ungewolltes Entweichen des Meßgases aus der Meßgaskammer in die Umgebung bei herausgenommenem Gassensor ist nicht mehr möglich.

Durch die besonders vorteilhafte Ausgestaltung der Sensorhalterung als eine Anlagevorrichtung wird auf einfache Weise die Zentrierung der gassensitiven Meßfläche des Gassensors über der Begasungsöffnung der Meßgaskammer ermöglicht. Das Druckstück ist dabei an die Anlagevorrichtung als Spannwippe angelenkt.

Es erweist sich als besonders zweckmäßig, die Spannwippe zweiarmig auszubilden und den einen Hebelarm mit einem gabelförmigen Endteil zu versehen, in welchen das Gehäuse des Gassensors aufgenommen werden kann. Der zweite Hebelarm kann dann als Betätigungsteil ausgebildet sein, wodurch eine Kopplung zwischen dem Einsetzen des Gassensors in die Einhängevorrichtung und der Betätigung des Absperrelementes erzielt wird.

In besonders einfacher Weise wird als Absperrelement ein über einen Stößel betätigbares Ventil gewählt, an dem der Betätigungsteil der zweiarmigen Spannwippe angreift. Wird somit der gabelförmige Endteil der Spannwippe angehoben, um den Gassensor aufzunehmen, wird gleichzeitig das Ventil über den Stößel geöffnet, und das Meßgas kann schon jetzt durch die Zuführungsleitung in die Meßgaskammer geleitet werden. Dadurch wird der stromabwärts liegende Teil der Zuführungsleitung sowie die Meßgaskammer selbst mit dem zu messenden Meß-bzw. Kalibriergas gespült. Wird die Spannwippe losgelassen, übt der gabelförmige Endteil als Druckstück einen Druck auf den in der Anlagevorrichtung befindlichen Gassensor aus, so daß dessen gassensitive Meßfläche auf der Begasungsöffnung der Meßgaskammer aufliegt.

Damit die Spannwippe ausschließlich bei herausgenommenem Gassensor das Ventil absperrt und nicht schon bei in die Einhängevorrichtung eingelegtem Gassensor, sind an der Innenseite der Gabelenden jeweils Haltenasen angebracht, die auf einem breiteren Bund des Gassensors aufruhen, aber an der schmaleren Anlagevorrichtung vorbeigleiten. Bei eingelegtem Gassensor halten diese den gabelförmigen Endteil so weit von der Begasungsöffnung der Meßgaskammer entfernt, daß der als Betätigungsteil ausgebildete zweite Hebelarm der Spannwippe den Stößel noch so weit betätigt, daß das Ventil geöffnet bleibt.

Ein Ausführungsbeispiel der Erfindung ist anhand der Zeichnung schematisch dargestellt und wird im folgenden näher beschrieben.

Es zeigen:

Figur I einen Querschnitt durch die Aufnahmevorrichtung mit eingelegtem Gassensor

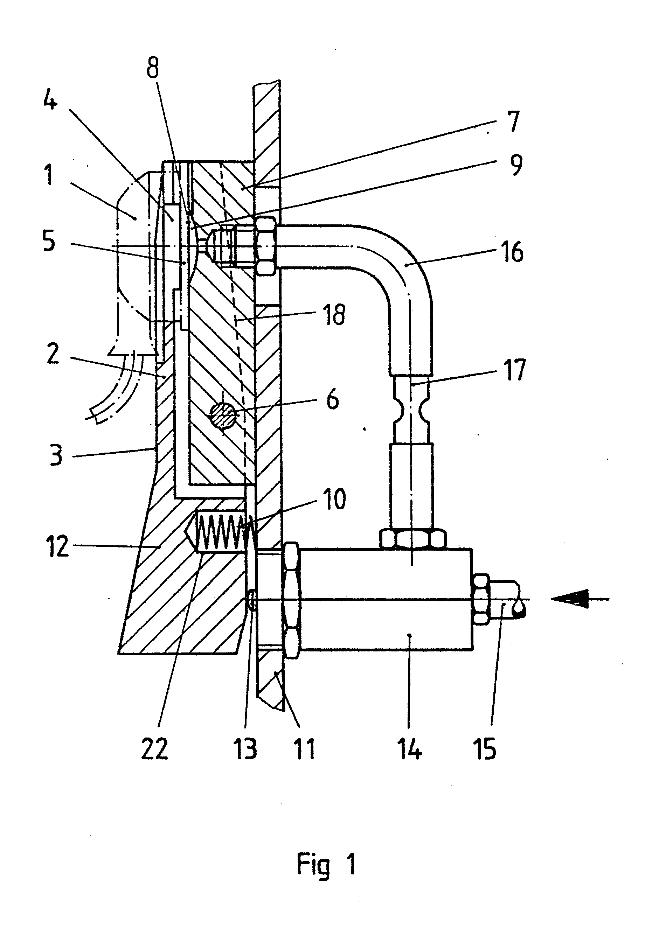

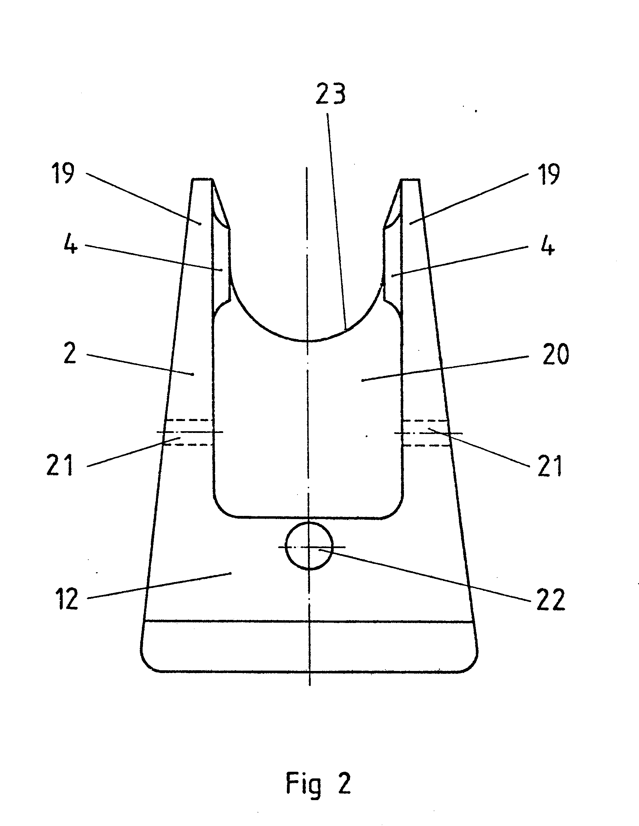

Figur 2 die Innenansicht der Spannwippe.

In Figur I befindet sich der Gassensor I zwischen den Gabelenden I9 des gabelförmigen Endteils 2 des als Spannwippe ausgebildeten Druckstückes 3. Die Haltenasen 4 stützen sich auf einen Bund 5 des Sensors I, der dadurch fest in das gabelförmige Endteil 2 eingespannt ist. Die mit dem Gelenk 6 an die Anlagevorrichtung 7 drehbar verbundene Spannwippe 3 drückt die gassensitive Meßfläche 8 des Sensors I gegen die Begasungsöffnung der Meßgaskammer 9. Die dazu notwendige Druckkraft wird durch eine Druckfeder I0 bewirkt, welche zwischen einer Montagewand II und dem Betätigungsteil I2 der Spannwippe angreift. Der Betätigungsteil I2 öffnet durch den eingedrückten Stößel I3 ein Ventil I4, wodurch eine Verbindung zwischen dem das Meßgas zuführenden Anschlußstutzen I5 und der Zuführungsleitung I6 bis in die Meßgaskammer 9 hergestellt ist. In der Zuführungsleitung I6 befindet sich eine Drossel I7.

In dem gezeigten Zustand strömt das Meßgas aus der nicht dargesellten Meßgasquelle durch das Ventil I4 über die Zuführungsleitung I6 in die Meßgaskammer 9 und kann dort über deren Begasungsöffnung an die gassensitive Meßfläche 8 und über vorhandene leichte Undichtigkeiten in die Atmosphäre entweichen. Wird der Gassensor I aus der Spannwippe 3 entnommen, liegt aufgrund der Wirkung der Druckfeder I0 das obere Ende der gestrichelt dargestellten Seitenfläche I8 des Druckstückes an der Montagewand II an, so daß der Stößel I3 so weit aus dem Ventil I4 herausragt, daß die Zufuhr von Meßgas über den Anschlußstutzen I5 unterbrochen ist.

Die in Figur 2 in der Innenansicht dargestellte Spannwippe 3 zeigt den gabelförmigen Endteil 2 mit den beiden Gabelenden I9, an deren Innenseiten die Haltenasen 4 angebracht sind. Die beiden Gabelenden I9 laufen in Richtung des Betätigungsteils I2 zu einem Zwischenstück 20 zusammen, welches eine den Gabelenden I9 zugewandte Kontur 23 aufweist, die den Gassensor I umgreift. Die Öffnungen 2I für die Aufnahme des Gelenks 6 sind gestrichelt dargestellt. Die Bohrung 22 ist für die Aufnahme der Druckfeder I0 bestimmt.

## Ansprüche

I. Aufnahmevorrichtung zum Anschluß von Gassensoren an eine mit einer Begasungsöffnung versehene Meßgaskammer, in welche eine ein betätigbares Absperrelement enthaltende Zuführungsleitung für das Meßgas mündet und an deren Begasungsöffnung eine Sensorhalterung angeordnet ist, an welche ein bewegliches, in Richtung auf die Begasungsöffnung wirkendes Druckstück angreift, **dadurch gekennzeichnet** , daß das Druckstück als Betätigungsteil (I2) für das Absperrelement (I4) der Zuführungsleitung (I6) ausgebildet ist.

2. Aufnahmevorrichtung nach Anspruch I, **dadurch gekennzeichnet,** daß die Sensorhalterung als eine die Begasungsöffnung umgebende Anlagevorrichtung (7) für den Sensor (I) ausgebildet ist, an welche das Druckstück als Spannwippe (I2) angelenkt ist.

3. Aufnahmevorrichtung nach Anspruch 2, **dadurch gekennzeichnet,** daß die Spannwippe -(I2) zweiarmig ist, und daß der erste Hebelarm als gabelförmiges Endteil (2) und der zweite Hebelarm als Betätigungsteil (I2) ausgebildet ist.

4. Aufnahmevorrichtung nach Anspruch 3, **dadurch gekennzeichnet,** daß das Absperrelement als ein über einen Stößel (I3) betätigbares Ventil (I4) ausgebildet ist, an den das Betätigungsteil (I2) angreift.

5. Aufnahmevorrichtung nach Anspruch 3, **dadurch gekennzeichnet,** daß an dem Endteil -(2) jeweils an der Innenseite seiner Gabelenden -(I9) Haltenasen (4) angebracht sind.

Fig 1

Fig 2